# EUROPEAN PATENT APPLICATION

(11) **EP 2 452 949 A1**
(43) Date of publication of application: **16.05.2012**
(21) Application number: 10796766.3
(22) Date of filing: 12.07.2010
(51) Int. Cl.: C07K 14/47, A61K 38/43, A61P 13/00

(54) **USE OF CARBONIC ANHYDRASE II FOR PRODUCING A DRUG**

(30) Priority: 10.07.2009 ES 200930437
(71) Applicant: Consejo Superior de Investigaciones Cientificas (CSIC), 28006 Madrid (ES)
(72) Inventor: HOTTER CORRIPIO, Georgina, E-08036 Barcelona (ES); VIÑAS, José Luis, E-50018 Zaragoza (ES); SOLA MARTÍNEZ, Anna, E-50018 Zaragoza (ES)
(74) Representative: Pons Ariño, Angel
(86) International application number: PCT/ES2010/070484
(87) International publication number: WO 2011/004054

(57) **Abstract**

The present invention relates to the field of biomedicine. Specifically, the present invention relates to the use of carbonic anhydrase II for producing a drug for preventing and/or treating damage caused by ischemia, ischemia followed by reperfusion or a toxin, acute failure or rejection of an organ transplant, preferably of a kidney. In a preferred embodiment, the toxin is cisplatin.

## Description

The present invention falls within the field of biomedicine. Specifically, the present invention relates to the use of carbonic anhydrase II for the manufacture of a medicament for the prevention and/or treatment of the damage caused by ischemia, ischemia followed by reperfusion or toxins, acute failure or rejection of a transplanted organ, preferably the kidney. In a preferred embodiment, the toxin is cisplatin.

### PRIOR STATE OF THE ART

Pathologies of ischemic origin are the main cause of death in developed countries. In the case of the kidney, acute renal failure (ARF) is a disease with a mortality of over 50%, a figure that has not undergone significant changes in the past 4 decades. In general, patients with clinical symptoms of renal failure are treated after the damage has developed, except in the case of patients who are to be subjected to renal transplantation. Since the disease is already developed when the patient arrives at the hospital, there is an urgent need for healing pathways through the stimulation of the regenerative and healing process in general.

The development of new and potent drugs has meant an important advance in the treatment of diseases that were lethal until recent times. However, some of these treatments are the cause of a high morbidity due to their toxicity. The consequence of the renal toxicity of these drugs and other toxic agents whereto patients may be accidentally exposed (heavy metal salts, hydrocarbons, vegetable or bacterial toxins) is the onset of ARF.

Cisplatin has been proven to be an effective chemotherapeutic agent, and is used in the treatment of a wide variety of neoplastic diseases. Unfortunately, treatment with cisplatin is associated with a high toxicity, which makes it necessary to reduce the dose or interrupt the treatment. One of the most significant adverse effects of cisplatin is nephrotoxicity; dose-dependent, cumulative renal failure is the main dose-limiting toxicity. Approximately 25% to 35% of patients develop nephrotoxicity following a single dose of cisplatin. Renal toxicity becomes more prolonged and severe after repeated treatment cycles; for this reason, it is essential to verify that the renal function has been normalised prior to beginning a new treatment with cisplatin. Nephrotoxicity may be prevented by maintaining adequate hydration before, during and after the intravenous perfusion of cisplatin. Forced diuresis by means of hydration or by means of hydration and the administration of a diuretic before and after the treatment with cisplatin reduces the risk of nephrotoxicity. However, nephrotoxicity may appear even when these processes are used.

The therapeutic measures used thus far for the regeneration of the damaged kidney have been: the application of growth factors (Hirschberg et al. Kidney lnt. 1999, 55: 2423-2432; Miller and Padanilam. Chapter 17: Molecular responses and growth factors, in: Atlas of diseases of the kidney, Robert W Schrier, pp. 17.1-17.16, Blackwell Science Press, Philadelphia, USA. 1999) or assays with stem cells (Brodsky et al. Am J Physiol Renal Physiol. 2002, 282: F1140-F1149; Kale et al. J Clin Invest. 2003, 112: 42-49; Lin F et al. J Am Soc Nephrol. 2003, 14: 1188-1199; Gupta et al. Kidney lnt. 2002, 62: 1285-1290; Oliver et al. Clin Invest. 2004, 114: 795-803), but the desired regenerative result has not been achieved. Consequently, there is a need for therapies that make it possible to reduce renal damage and enhance regeneration.

### EXPLANATION OF THE INVENTION

The present invention relates to the use of carbonic anhydrase II for the manufacture of a medicament for the prevention and/or treatment of the damage caused by ischemia, ischemia followed by reperfusion or toxins, acute failure or rejection of a transplanted organ, preferably the kidney. In a preferred embodiment, the toxin is cisplatin.

Carbonic anhydrase is a metalloenzyme that is widely distributed throughout the entire body (renal cortex, glial tissues, the eye, erythrocytes, etc.) and reversibly catalyses the conversion of carbonic anhydride and water into carbonic acid. This enzyme presents four isoforms. Amongst them, carbonic anhydrase II (CAII) is the most active isoenzyme and its amino acid sequence in humans is SEQ ID NO: 1 (*Genbank* reference number: NP_000058).

The examples of the present patent application demonstrate the capacity of CAII to induce regeneration and inhibit apoptosis in both an *in vivo* experimental model of renal ischemia-reperfusion and in an *in vitro* experimental model of damage induced by the toxin cisplatin. These analyses show the utility of CAII in preventing and/or treating a tissue or an organ.

Therefore, a first aspect of the present invention relates to the use of a protein that comprises amino acid sequence SEQ ID NO: 1, a variant thereof or of a fragment of the above for the manufacture of a medicament.

The terms "protein", "amino acid sequence", "polypeptide" and "peptide" are used interchangeably herein, and refer to a polymeric form of amino acids of any length, which may or may not be chemically or biochemically modified.

In the sense used in this description, the term "variant" refers to a protein that is substantially homologous and functionally equivalent to the protein that comprises amino acid sequence SEQ ID NO: 1. In general, a variant includes additions, deletions or substitutions of amino acids that do not substantially alter the function thereof. The term "variant" also includes those proteins resulting from post-translational modifications, such as, for example, without being limited thereto, glycosylation or phosphorylation.

As used herein, a protein is substantially homologous to the protein that comprises amino acid sequence SEQ ID NO: 1 when its amino acid sequence has a degree of identity with respect to the amino acid sequence of said protein of, at least, 60%, advantageously of, at least, 70%, preferably of, at least, 80%, more preferably of, at least, 90%, and more preferably of at least 95%.

The term "identity", as used in the present description, refers to the proportion of identical amino acids between two amino acid sequences that are being compared. The identity percentage between two sequences may be easily identified by a person skilled in the art, for example, with the aid of an appropriate computer programme for comparing sequences.

The expression "functionally equivalent", as used in the present description, means that the protein in question maintains the capacity to induce regeneration. Said capacity may be determined by means of conventional methods, such as the assays described in Example 1 that goes with this description.

Likewise, in the sense used in this description, the term "fragment" refers to a peptide that comprises a portion of the protein which comprises amino acid sequence SEQ ID NO: 1, i.e. a sequence of contiguous amino acids included within said SEQ ID NO: 1; or a peptide that comprises a portion of a variant of the protein that comprises amino acid sequence SEQ ID NO: 1; provided that said fragment or peptide is functionally equivalent.

The protein that comprises amino acid sequence SEQ ID NO: 1, the variant thereof or the fragment of the above may be obtained by conventional methods known in the state of the art.

A second aspect of the present invention relates to the use of a polynucleotide (hereinafter, polynucleotide of the invention) which encodes the protein that comprises amino acid sequence SEQ ID NO: 1, a variant thereof or a fragment of the above, for the manufacture of a medicament.

The terms "polynucleotide", "nucleic acid" and "nucleotide sequence" are used interchangeably herein, abd refer to polymeric forms of nucleotides of any length, both ribonucleotides (RNA) and deoxyribonucleotides (DNA).

In a preferred embodiment of this second aspect, the polynucleotide of the invention comprises nucleotide sequence SEQ ID NO: 2, which corresponds to the nucleotide sequence of the cDNA of the human CAII protein *(Genbank* reference number: NG_007287).

A third aspect of the invention relates to the use of a gene construct (hereinafter, gene construct of the invention) that comprises the polynucleotide of the invention for the manufacture of a medicament.

The gene construct of the invention may comprise the polynucleotide of the invention, operatively linked to a regulatory sequence for the expression of the polynucleotide of the invention, thereby constituting an expression cassette.

"Operatively linked" refers to a juxtaposition wherein the components thus described have a relationship that makes it possible for them to function in the intended manner. A control sequence "operatively linked" to the polynucleotide is linked thereto in such a way that the expression of the polynucleotide coding sequence is achieved.

"Control sequences" refer to polynucleotide sequences that affect the expression of the sequences whereto they are linked. Said control sequences include, for example, without being limited thereto, promoters, initiation signals, termination signals, enhancers and silencers. The term "control sequences" is intended to include, at least, all the components the presence whereof is necessary for expression, and may also include additional components the presence whereof is advantageous.

In a preferred embodiment of this second aspect, the gene construct of the invention comprises the polynucleotide of the invention operatively linked to, at least, one control sequence from the list that comprises:
a. a promoter that directs the transcription of said polynucleotide,
b. a transcription initiation signal,
c. a transcription termination signal,
d. a polyadenylation signal, or
e. a transcriptional activator.

As used herein, the term "promoter" refers to a DNA region located at the 5' position with respect to the transcription initiation point which is necessary for or facilitates said transcription in an animal cell. This term includes, for example, without being limited thereto, constitutive promoters, cell- or tissue-type-specific promoters, and inducible or repressible promoters.

In a particular embodiment, the expression control sequences are functional in prokaryotic cells and organisms, for example, without being limited thereto, bacteria; whereas in another particular embodiment, said expression control sequences are functional in eukaryotic cells and organisms, for example, animal cells. Preferably, the control sequences are functional in mammal cells and, more preferably, in human cells.

The polynucleotide of the invention or the gene construct of the invention may be introduced into the interior of a cell, called host cell, for example, without being limited thereto, as a naked nucleic acid or by means of a vector.

A fourth aspect of the invention relates to the use of a vector (hereinafter, vector of the invention) that comprises the polynucleotide of the invention or the gene construct of the invention, for the manufacture of a medicament.

As used in the present invention, the term "vector" refers to a system used to introduce an exogenous nucleic acid into the interior of a prokaryotic or eukaryotic cell, thereby allowing for vehiculisation of the nucleic acid into the interior of the cell.

There are numerous viral and non-viral viral vectors known in the state of the art. Viral vectors include, without being limited thereto, the following: adenoviral vectors, adeno-associated vectors, retroviral vectors, lentiviral vectors, alphavirus vectors, herpesvirus vectors and coronavirus-derived vectors. Non-viral type vectors include, without being limited thereto, the following: gene gun, liposomes, polyamines, peptides, dendrimers, cationic glycopolymers, liposome-polycation complexes, proteins and receptor-mediated gene transfer systems.

In an even more preferred embodiment of this aspect of the invention, the vector is a gene expression vector. The term "expression vector", as used in the present description, refers to a nucleic acid molecule wherein another nucleic acid molecule may be integrated without it losing its self-replication capacity and which, moreover, is adequate to express said nucleic acid integrated therein after being introduced into a host cell. The choice of the vector will be dependent on the host cell wherein it will be subsequently introduced.

A fifth aspect of the invention relates to a host cell (hereinafter, cell of the invention) that comprises the polynucleotide of the invention, the gene construct of the invention or the vector of the invention, and which is capable of expressing the protein of the invention.

The cell of the invention may be prokaryotic or eukaryotic. Preferably, the cell of the invention is an animal cell, more preferably, a mammal cell, and, even more preferably, a human cell.

The polynucleotide of the invention, the gene construct of the invention, the vector of the invention and the cell of the invention may be obtained by conventional methods known in the state of the art.

A preferred embodiment of the present invention relates to the use of the protein that comprises amino acid sequence SEQ ID NO: 1, the variant thereof or the fragment of the above, the polynucleotide of the invention, the gene construct of the invention, the vector of the invention or the cell of the invention for the manufacture of a medicament for the prevention and/or treatment of a damage in a tissue or an organ.

A more preferred embodiment of the present invention relates to the use of the protein that comprises amino acid sequence SEQ ID NO: 1, the variant thereof or the fragment of the above, the polynucleotide of the invention, the gene construct of the invention, the vector of the invention or the cell of the invention for the manufacture of a medicament for the prevention and/or treatment of a damage caused by ischemia, ischemia-reperfusion or toxins in a tissue or an organ.

The term "ischemia" refers to a transient or permanent reduction in the blood flow in a tissue or organ, with the consequent reduction in the supply of oxygen. The set of damages undergone by the tissue or organ due to ischemia is known as "damage caused by ischemia".

The term "reperfusion" refers to the restoration of the blood supply to a tissue or an organ that is ischemic as a consequence of a reduction in the normal blood flow. The recovery of the blood flow restores the supply of oxygen and nutrients to the tissue, thereby allowing for the recovery of the ischemic tissue or organ. However, reperfusion by itself may injure the ischemic tissue or organ, leading to what is known as "damage caused by reperfusion".

The expression "damage caused by ischemia-reperfusion" refers to the set of damages undergone by a tissue or an organ due to a reduction in the blood flow (ischemia) followed by a restoration of the blood flow (reperfusion).

The expression "damage caused by toxins" refers to the set of damages undergone by a tissue or an organ as a consequence of the exposure thereof to one or more toxins, such as, for example, without being limited thereto, an antibiotic, an anaesthetic, a chemotherapeutic agent, a radiological contrast, a heavy metal, a fungicide, a pesticide, an organic solvent, an animal poison, a fungal toxic agent or a toxic agent of endogenous origin.

Cisplatin (cis-diamino-dichloro-platinum II) has proven to be an effective chemotherapeutic agent, and is used in the treatment of a wide variety of neoplastic diseases. Unfortunately, the exposure of patients to cisplatin is associated with nephrotoxicity, neurotoxicity, ototoxicity, gastrointestinal toxicity, cardiotoxicity and hepatotoxicity, amongst other secondary effects.

A preferred embodiment of the present invention relates to the use of the protein that comprises amino acid sequence SEQ ID NO: 1, the variant thereof or the fragment of the above, the polynucleotide of the invention, the gene construct of the invention, the vector of the invention or the cell of the invention for the manufacture of a medicament for the prevention and/or treatment of a damage caused by cisplatin in a tissue or an organ.

In a preferred embodiment of the present invention, the tissue is an epithelium. An animal epithelium is a tissue formed by one or several layers of cells in close contact, which coats the surface, the cavities and the ducts of the body, the epidermis and the outer layer of mucous membranes, and the secretory portion of glands. In a more preferred embodiment, the epithelium is selected from the list that comprises: renal epithelium, hepatic epithelium, pulmonary epithelium, gastric epithelium, intestinal epithelium, auditory epithelium, pancreatic epithelium, urinary transitional epithelium, uterine epithelium and skin epidermis. In an even more preferred embodiment, the epithelium is the renal epithelium.

In a preferred embodiment of the present invention, the organ is selected from the list that comprises: kidney, liver, brain, heart, lung, stomach, intestine, ear, pancreas, bladder, uterus and skin. In a more preferred embodiment, the organ is the kidney.

Renal ischemia consists of a transient or permanent reduction in the blood flow, with the consequent reduction in the supply of oxygen to the kidney, as a consequence, for example, without being limited thereto, of a reduction in the total blood volume, a redistribution of the blood or an obstruction. The reduction in the blood flow may be unilateral, when it affects only one kidney, or bilateral, when it affects both kidneys. The set of damages undergone by the renal epithelium or the kidney due to ischemia is known as "damage caused by renal ischemia".

A preferred embodiment of the present invention relates to the use of the protein that comprises amino acid sequence SEQ ID NO: 1, the variant thereof or the fragment of the above, the polynucleotide of the invention, the gene construct of the invention, the vector of the invention or the cell of the invention for the manufacture of a medicament for the prevention and/or treatment of the damage caused by ischemia in the renal epithelium or the kidney, i.e. the damage caused by renal ischemia.

The expression "damage caused by renal ischemia-reperfusion" refers to the set of damages undergone by the kidney due to a reduction in the blood flow (renal ischemia) followed by a restoration of the blood flow (reperfusion).

A preferred embodiment of the present invention relates to the use of the protein that comprises amino acid sequence SEQ ID NO: 1, the variant thereof or the fragment of the above, the polynucleotide of the invention, the gene construct of the invention, the vector of the invention or the cell of the invention for the manufacture of a medicament for the prevention and/or treatment of the damage caused by ischemia-reperfusion in the renal epithelium or the kidney, i.e. the damage caused by renal ischemia-reperfusion.

A preferred embodiment of the present invention relates to the use of the protein that comprises amino acid sequence SEQ ID NO: 1, the variant thereof or the fragment of the above, the polynucleotide of the invention, the gene construct of the invention, the vector of the invention or the cell of the invention for the manufacture of a medicament for the prevention and/or treatment of the damage undergone by the renal epithelium or the kidney as a result of toxins such as, for example, without being limited thereto, an antibiotic, an anaesthetic, a chemotherapeutic agent, a radiological contrast, a heavy metal, a fungicide, a pesticide, an organic solvent, an animal poison, a fungal toxic agent or a toxic agent of endogenous origin.

One of the most significant adverse effects of treatment with the chemotherapeutic agent cisplatin is nephrotoxicity. In fact, dose-dependent, cumulative renal failure is the main dose-limiting toxicity. Approximately 25% to 35% of patients develop nephrotoxicity after a single dose of cisplatin. The most commonly observed changes are a reduction in the glomerular filtration rate, which is reflected in an increase in the serum creatinine and a reduction in the effective renal plasma flow. Renal toxicity becomes more prolonged and severe following repeated treatment cycles; for this reason, it is essential to verify that the renal function has been normalised prior to beginning a new treatment with cisplatin.

A preferred embodiment of the present invention relates to the use of the protein that comprises amino acid sequence SEQ ID NO: 1, the variant thereof or the fragment of the above, the polynucleotide of the invention, the gene construct of the invention, the vector of the invention or the cell of the invention for the manufacture of a medicament for the prevention and/or treatment of the damage undergone by the renal epithelium or the kidney as a consequence of the exposure thereof to cisplatin, i.e. the renal damage caused by cisplatin.

The onset of nephrotoxicity caused by cisplatin may be intensified by the concomitant treatment with antihypertensives that contain, for example, without being limited thereto, furosemide, hydralazine, diazoxide and propranolol. The concomitant administration of other medicaments such as, for example, without being limited thereto, cephalosporins, aminoglycosides or Amphotericin B, or contrast media, enhances the nephrotoxic effects of cisplatin. Therefore, the present invention is useful for the prevention and/or treatment of a damage caused by the exposure of the renal epithelium or the kidney to cisplatin combined with another or other toxins, such as, for example, without being limited thereto, those mentioned above.

As a consequence of the damages caused by ischemia, ischemiareperfusion or toxins in an organ, acute organ failure may occur. The terms "acute failure" and "acute organ failure" refer to a clinical syndrome that is characterised by an abrupt deterioration of the function of a given organ.

A preferred embodiment of the present invention relates to the use of the protein that comprises amino acid sequence SEQ ID NO: 1, the variant thereof or the fragment of the above, the polynucleotide of the invention, the gene construct of the invention, the vector of the invention or the cell of the invention for the manufacture of a medicament for the prevention and/or treatment of an acute failure of an organ caused by ischemia, ischemia-reperfusion or toxins. In a preferred embodiment of the present invention, the organ is selected from the list that comprises: kidney, liver, brain, heart, lung, stomach, intestine, pancreas, bladder, uterus and skin. In a more preferred embodiment, the organ is the kidney.

As a consequence of a damage caused by ischemia, ischemia-reperfusion or toxins in the kidney, acute renal failure (ARF) may occur. The terms "acute renal failure", "acute renal malfunction" or "acute reanl insufficiency" (ARI) refer to a clinical syndrome characterised by an abrupt deterioration of the renal function, which causes a reduction in glomerular filtration and an accumulation of serum nitrogen products (such as, for example, urea or creatinine), and alterations of the hydroelectrolytic balance and the acid-base balance may also occur. ARF may be classified into three large groups: functional ARF, parenchymatous ARF and obstructive ARF. In functional ARF, there is inadequate renal perfusion, which compromises glomerular filtration, but the glomerular parenchyma remains intact. Renal failure that takes place during functional ARF is reversible upon restoring the plasma flow, but, if the situation that triggered it persists, it will evolve towards parenchymatous ARF. In parenchymatous ARF, the cause of the deterioration of the renal function is a damage in the different kidney anatomical structures, which leads to different clinical syndromes: the tubule (acute tubular necrosis), the glomerulus (glomerular necrosis), the tubular interstice (tubulo-interstitial necrosis) or the blood vessels. In obstructive ARF, there is an increase in the pressure in the urinary tract, which is transmitted retrogradely, thereby compromising normal glomerular filtration, as a consequence of the obstruction of any of the kidney ducts.

A preferred embodiment of the present invention relates to the use of the protein that comprises amino acid sequence SEQ ID NO: 1, the variant thereof or the fragment of the above, the polynucleotide of the invention, the gene construct of the invention, the vector of the invention or the cell of the invention for the manufacture of a medicament for the prevention and/or treatment of an ARF caused by ischemia, ischemia-reperfusion or toxins.

A preferred embodiment of the present invention relates to the use of the protein that comprises amino acid sequence SEQ ID NO: 1, the variant thereof or the fragment of the above, the polynucleotide of the invention, the gene construct of the invention, the vector of the invention or the cell of the invention for the manufacture of a medicament for the prevention and/or treatment of an ARF caused by exposure of the renal epithelium or the kidney to cisplatin.

One of the most frequent damage situations caused by ischemia-reperfusion takes place in organ transplantation. In fact, acute organ failure is one of the main causes of the rejection of transplanted organs.

A preferred embodiment of the present invention relates to the use of the protein that comprises amino acid sequence SEQ ID NO: 1, the variant thereof or the fragment of the above, the polynucleotide of the invention, the gene construct of the invention, the vector of the invention or the cell of the invention for the manufacture of a medicament for the prevention and/or treatment of the rejection of a transplanted organ. In a preferred embodiment of the present invention, the organ is selected from the list that comprises: kidney, liver, brain, heart, lung, stomach, intestine, ear, pancreas, bladder, uterus and skin. In a more preferred embodiment, the organ is the kidney.

ARF associated with the damage caused by ischemia-reperfusion is one of the main causes of the initial delay in the function or the rejection of a transplanted kidney.

A preferred embodiment of the present invention relates to the use of the protein that comprises amino acid sequence SEQ ID NO: 1, the variant thereof or the fragment of the above, the polynucleotide of the invention, the gene construct of the invention, the vector of the invention or the cell of the invention for the manufacture of a medicament for the prevention and/or treatment of the rejection of a transplanted kidney.

Another aspect of the invention relates to a pharmaceutical composition (hereinafter, pharmaceutical composition of the invention) that comprises the protein which comprises SEQ ID NO: 1, a variant thereof or a fragment of the above, the polypeptide of the invention, the gene construct of the invention or the cell of the invention.

A preferred embodiment of this aspect relates to the use of the pharmaceutical composition of the invention for the prevention and/or treatment of a damage in a tissue or an organ. A more preferred embodiment relates to the use of the pharmaceutical composition of the invention for the prevention and/or treatment of a damage caused by ischemia, ischemia-reperfusion or toxins in a tissue or an organ. An even more preferred embodiment of this aspect relates to the use of the pharmaceutical composition of the invention for the prevention and/or treatment of a damage caused by cisplatin in a tissue or an organ. In a preferred embodiment, the tissue is an epithelium. In a more preferred embodiment, the epithelium is selected from the list that comprises: renal epithelium, hepatic epithelium, pulmonary epithelium, gastric epithelium, intestinal epithelium, auditory epithelium, pancreatic epithelium, urinary transitional epithelium, uterine epithelium and skin epidermis. In an even more preferred embodiment, the epithelium is the renal epithelium. In a preferred embodiment, the organ is selected from the list that comprises: kidney, liver, brain, heart, lung, stomach, intestine, ear, pancreas, bladder, uterus and skin. In a more preferred embodiment, the organ is the kidney.

A preferred embodiment of this aspect relates to the use of the pharmaceutical composition of the invention for the prevention and/or treatment of an acute failure of an organ caused by ischemia, ischemia-reperfusion or toxins. Preferably, the organ is selected from the list that comprises: kidney, liver, brain, heart, lung, stomach, intestine, ear, pancreas, bladder, uterus and skin. More preferably, the organ is the kidney.

A more preferred embodiment of this aspect relates to the use of the pharmaceutical composition of the invention for the prevention and/or treatment of ARF caused by ischemia, ischemia-reperfusion or toxins.

A preferred embodiment of this aspect relates to the use of the pharmaceutical composition of the invention for the prevention and/or treatment of an ARF caused by exposure of the renal epithelium or the kidney to cisplatin.

Another preferred embodiment of this aspect relates to the use of the pharmaceutical composition of the invention for the prevention and/or treatment of the rejection of a transplanted organ. Preferably, the organ is selected from the list that comprises: kidney, liver, brain, heart, lung, stomach, intestine, ear, pancreas, bladder, uterus and skin. More preferably, the organ is the kidney.

A more preferred embodiment of this aspect relates to the use of the pharmaceutical composition of the invention for the prevention and/or treatment of the rejection of a transplanted kidney.

In a preferred embodiment of this aspect, the pharmaceutical composition of the invention further comprises a pharmaceutically acceptable vehicle. In a more preferred embodiment of this aspect, the pharmaceutical composition of the invention further comprises another active principle. In a more preferred embodiment of this aspect, the pharmaceutical composition further comprises, jointly with a pharmaceutically acceptable vehicle, another active principle.

As used herein, the terms "active principle", "active substance", "pharmaceutically active substance", "active ingredient" and "pharmaceutically active ingredient" refer to any component that potentially provides pharmacological activity or a different effect in the diagnosis, cure, alleviation, treatment or prevention of a disease, or which affects the structure or function of the body of a human being or other animals.

The pharmaceutical composition of the invention may be formulated to be administered in a variety of forms known in the state of the art. Such formulations may be administered to an animal and, more preferably, to a mammal, including a human being, by a variety of routes, including, without being limited thereto, parenteral, intraperitoneal, intravenous, intradermal, epidural, intraspinal, intrastromal, intra-articular, intrasinovial, intrathecal, intralesional, intra-arterial, intracapsular, intracardiac, intramuscular, intranasal, intracraneal, subcutaneous, intraorbital, intracapsular or topical.

The dosing necessary to obtain a therapeutically effective amount is dependent on a variety of factors, such as, for example, the age, weight, sex or tolerance of the animal. In the sense used in this description, the expression "therapeutically effective amount" refers to the amount of the pharmaceutically effective composition that produces the desired effect and, in general, will be determined, amongst other factors, by the characteristics of said pharmaceutical composition and the therapeutic effect to be achieved. The pharmaceutically acceptable "adjuvants" or "vehicles" that may be used in said compositions are the vehicles known in the state of the art.

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practise of the invention. The following figures and examples are provided for illustrative purposes, and are not intended to limit the scope of the present invention.

### DESCRIPTION OF THE FIGURES

Figure 1. Shows the *in vivo* analysis of the effect of CAII on renal regeneration. A. Effect of CAII on the expression of the PCNA proliferation and regeneration marker. B. Effect of CAII on the expression of the Stathmin proliferation and regeneration marker. Data represented as the mean +/- SEM; n=5; * p<0.05 vs I/R; + p<0.05 vs I/R + CAII.
Figure 2. Shows the *in vivo* analysis of the effect of CAII on the incidence of apoptosis in renal damage, evaluated by measuring the activity of caspase 3. Data represented as the mean +/- SEM; p<0.05; n=5; * p<0.05 vs control; + p<0.05 vs I/R.
Figure 3. Shows the *in vivo* analysis of the effect of CAII on the expression of Erythropoietin (EPO). Data represented as the mean +/- SEM; p<0.05; n=5; * p<0.05 vs control; + p<0.05 vs I/R.
Figure 4. Shows the *in vitro* analysis of the effect of CAII on renal regeneration. A. Effect of CAII on the expression of the PCNA proliferation and regeneration marker. B. Effect of CAII on the expression of the Ki67 proliferation and regeneration marker. Data represented as the mean +/- SEM; p<0.05; n=5; * p<0.05 vs cis; + p<0.05 vs cis + CAII.
Figure 5. Shows the *in vitro* analysis of the effect of CAII on the incidence of apoptosis in renal damage, evaluated by measuring the activity of caspase 3. Data represented as the mean +/- SEM; p<0.05; n=5; * p<0.05 vs control; + p<0.05 vs cisplatin.

### EXAMPLES

The following specific examples provided in this patent document serve to illustrate the nature of the present invention. These examples are included solely for illustrative purposes and should not be interpreted to limit the invention claimed herein. Therefore, the examples described further below illustrate the invention without limiting the field of application thereof.

### EXAMPLE 1. IN VIVO ANALYSIS OF THE EFFECT OF CAII ON CELL REGENERATION AND DAMAGE IN THE RENAL LESION INDUCED BY ISCHEMIA-REPERFUSION

### MATERIALS AND METHODS USED:

### In vivo model of renal ischemia-reperfusion

Swiss strain mice were used, males with an approximate weight of 25-30 g (Charles River, France). All the procedures were performed under the supervision of the ethics committee of the Institute for Biomedical Research of Barcelona (CSIC) and followed European Union guidelines. The environmental conditions were kept constant, the temperature was 21-22°C, the relative humidity was 70% and the alternative cycles of light/darkness were 12 h. The animals were fed a standard diet of AO4 fodder (Panlab, Barcelona) and water from the Barcelona supply network *ad libitum.*

The animals were anaesthesised with lsoflurane, placed in the supine position, and their body temperature was maintained at between 36°C and 37°C. After performing a median laparotomy to access the kidney, carefully putting aside the intestinal packet, bilateral ischemia was induced by clamping both renal arterio-venous pedicles with a non-traumatic microvascular clamp for 45 minutes. Subsequently, the reperfusion period was started, with removal of the clamp, and it was visually verified by observing the return of the blood flow to the kidney. Subsequently, the animal was sutured and administered subcutaneous Buprex (4.16 µg/100 g of weight).

Animals subjected to a sham operation were used as controls. During the entire operation process, the animals were well hydrated and the body temperature was maintained at about 37°C. During the reperfusion time, the animals were stabulated under the control of a veterinary. After 24 h from reperfusion, the animal was sacrificed for extraction of the kidneys and blood. The tissue was immediately frozen in carbonic snow and, subsequently, stored at -80°C.

### Study groups of the in vivo model of renal ischemia and reperfusion

I/R.- Animals subjected to 45 minutes of ischemia and 24 hours of reperfusion.

I/R + CAII.- Animals subjected to I/R, but with injection of CAII of human origin (Sigma, reference number: C-6165) at a concentration of 5 mg/kg at the beginning of reperfusion, by direct puncture of the inferior vena cava.

CONTROL.- Control animals, not subjected to ischemia/reperfusion.

CONTROL + CAII. Animals identical to those in the control group, but with intravenous injection of CAII at a concentration of 5 mg/kg 24 hours prior to collection of the samples.

I/R + Azt: Animals subjected to I/R, but with intraperitoneal injection of Azetazolamide (Sigma, reference number: A6011) at a concentration of 30 mg/kg one day prior to subjecting the animals to the I/R process.

### Real-time RT-PCR

The RNA of the kidney samples was extracted by means of the TRlzol reagent in accordance with the manufacturer's instructions (Invitrogen, Barcelona). Subsequently, the total RNA in the samples was purified using the RNeasy mini Kit from Qiagen (Madrid) in accordance with the manufacturer's instructions. The RNA concentrations were calculated by determining the absorbance at 260 nm. The integrity of the RNA thus obtained was examined by analysing the 18s and 28s ribosomal RNA bands detected and analysed in the Bioanalyzer of the Clinical Hospital of Barcelona (Agilent).

The expression of the analysed genes was measured by means of real-time quantitative RT-PCR normalised with the glyceraldehyde 3-phosphate dehydrogenase (GAPDH) housekeeping gene. Real-time RT-PCRs were performed in a Bio-Rad Thermal Cycler (iCycler iQ Real-Time PCR Detection System, Bio-Rad, Barcelona) and the amplifications were performed in 20 µl reactions using the two-step RT-PCR Kit with SYBR-Green (Bio-Rad), according to the manufacturer's instructions. The primers used were the following: *PCNA*: forward, SEQ ID NO: 3; reverse, SEQ ID NO: 4; *Stathmin:* forward, SEQ ID NO: 5; reverse, SEQ ID NO: 6; *GAPDH:* forward, SEQ ID NO: 7; reverse, SEQ ID NO: 8.

### Activity of Caspase 3

The activity of caspase-3 was determined by measuring the proteolysis of the specific substrate (N-acetyl-Asp-Glu-Val-Asp-7-amino-4-methyl coumarin (DEVD-AMC; Biomol, Plymouth Meeting, PA). The renal tissue samples were homogenised and sonicated in the assay buffer (50 mM HEPES, 10% sucrose, 0.1 % CHAPS, 5 mM GSSG, 5 mM DTT). The supernatants of the samples were incubated in this buffer, whereto DEVD-AMC at a concentration of 50 µM was added. The AMC released was quantified for 1 hour and a half at 37°C by fluorospectrophotometry, using 380 nm of excitation and measuring the emission at 450 nm.

### Western blot

The renal tissue samples were homogenised in lysis buffer (70 mM sucrose, 2 mM Hepes KOH, 220 mM Mannitol, 0.1 mM EGTA, 1 mM PMSF, 0.1 mM BSA, antiproteases cocktail (SIGMA)); subsequently, the protein concentration thereof was determined. The proteins were determined by means of the Bradford colorimetric method with a commercial reagent from BioRad.

50 µg of protein for each of the groups to be analysed were placed in each well of the 12% acrylamide gel. Following the electrophoresis, the proteins were transferred to nitrocellulose membranes, where they were subsequently blocked with 5% skim milk dissolved in buffered saline (TTBS) with 0.06% of Tween detergent. The nitrocellulose membranes were incubated overnight at 4°C with an anti-EPO antibody (Santa Cruz Antibodies, USA, sc-7956) at a 1/1000 dilution.

On the following day, the membranes were subjected to 3 washings, 5 minutes each, with TTBS, and were subsequently incubated with a peroxidase-conjugated anti-rabbit secondary antibody. After the end of the incubation period with the second antibody, and following three five-minute washings with TTBS, the detection process with ECL was performed.

### Statistical analysis

The data are shown as the mean +/- standard error from the mean (SEM), and values of p>0.05 were considered to be significant. The statistical differences between the groups were analysed by means of variance analysis (ANOVA) and, in the event of significance, Student's t-test was applied.

### RESULTS OBTAINED:

### In vivo analysis of the effect of CAII on renal regeneration

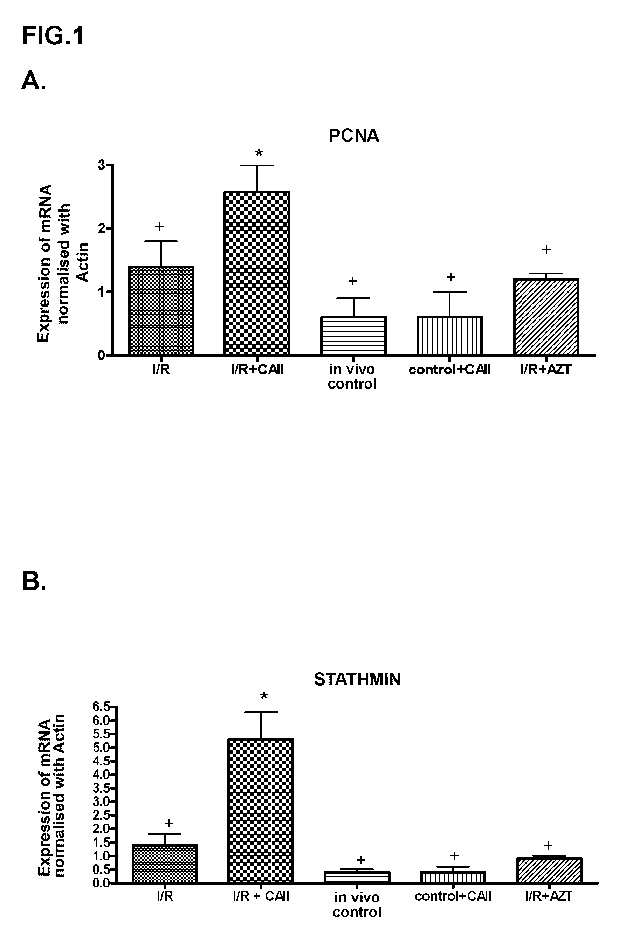

Figure 1 shows the results of the analysis of the messenger RNA of the PCNA and Stathmin genes; both are cell proliferation and regeneration markers. The samples were taken from mice subjected to the different treatments applied to the *in vivo* part of the experimental model of renal ischemia-reperfusion (I/R). As may be observed in Figure 1, the I/R process causes a regenerative response that is determined by an increase in the expression of both PCNA and Stathmin in the I/R groups, as compared to the control group. Treatment with CAII causes a marked increase in this regenerative response. This demonstrates the role of this protein as a regeneration promoter.

The administration of an inhibitor of CAII, azetazolamide (I/R + AZT), causes a decrease in the regenerative markers.

On the other hand, CAII showed a regenerative effect only on the damaged tissue, since the administration thereof to controls (control + CAII) did not induce regeneration.

### In vivo analysis of the effect of CAII on apoptosis in renal damage

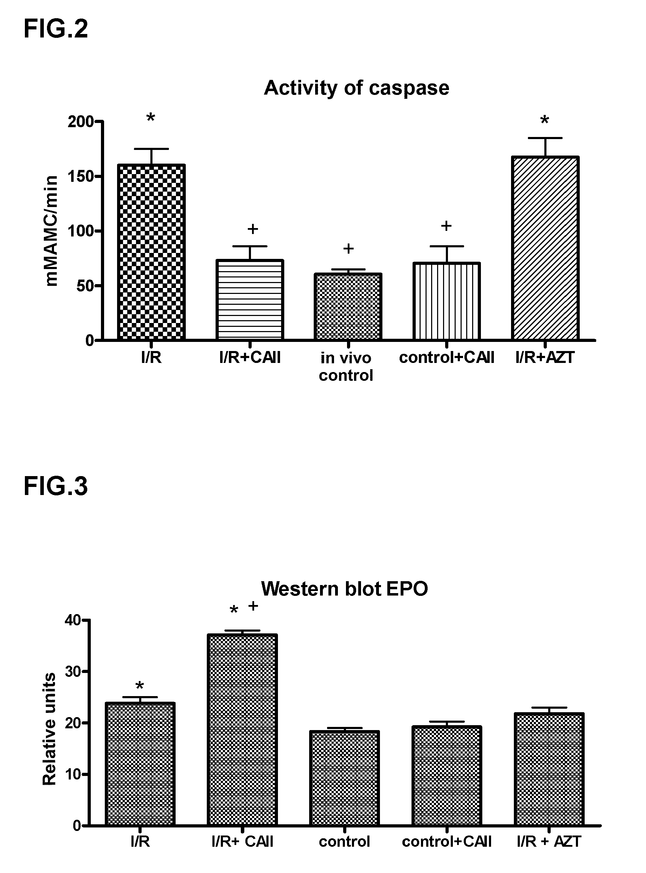

Figure 2 represents the cell damage, determined by the incidence of apoptosis and evaluated by the activity of caspase 3. We may observe that I/R induces a significant increase in this parameter as compared to the control group. This incidence of apoptosis is drastically reduced following treatment with CAII (I/R + CAII). The administration of CAII to control animals shows that CAII does not affect the apoptosis that is naturally present in healthy tissue (control group + CAII).

### In vivo analysis of the effect of CAII on the expression of Erythropoietin (EPO)

In an attempt to analyse in greater depth the mechanism whereby CAII exerts its regenerative and protective function against renal damage, we studied, *in vivo,* the relationship between CAII and Erythropoietin, with the hypothesis of a possible induction of EPO mediated by CAII.

The Western Blot for EPO (Figure 3) confirmed this possible relationship, since a marked increase in the amount of EPO present in the I/R group was observed when CAII was added; moreover, the inhibition of CAII by means of azetazolamide reduced the amount of EPO present in the kidney of the mice subjected to I/R (I/R + AZT), even below the levels present in the group subjected only to I/R.

### EXAMPLE 2. IN VITRO ANALYSIS OF THE EFFECT OF CAII ON THE REGENERATION OF THE RENAL DAMAGE INDUCED BY THE TOXIN CISPLATIN

### MATERIALS AND METHODS USED:

### Cell culture of renal tubular cells (in vitro study model)

Proximal tubular epithelial cells from rats (NRK52e) were kept in culture bottles (75 cm²), with a culture medium (DMEM) whereto 17.5 mM of glucose and 2.5 mM of glutamine were added; this medium was also supplemented with antibiotics (100 units/ml of penicillin and 100 µg/ml of streptomycin) and 10% foetal calf serum (FCS). The cells were incubated in a humidified atmosphere with 5% CO₂ at 37°C.

The control cells (control) were collected when they reached confluence without any treatment.

Treatment with cisplatin (Sigma) was performed by dissolving the drug in the minimum volume of DMSO (40 µl) and diluting it in PBS at a concentration of 200 µM. After 6 hours, the medium was removed and a new medium was added, without the drug for the **CIS** group and with 10 µg/ml of CAII for the **CIS + CAII** group. The samples were collected 16 hours later.

In the **control + CAII** group, the cells were treated with an identical dose of CAII for 16 hours until they were collected.

### Real-time RT-PCR

The RNA of the samples taken from the cell culture was extracted using the TRlzol reagent in accordance with the manufacturer's instructions (Invitrogen, Barcelona). Subsequently, the total RNA in the cells was purified using the RNeasy mini Kit from Qiagen (Madrid) according to the manufacturer's instructions. The RNA concentrations were calculated by determining the absorbance at 260 nm. The integrity of the RNA thus obtained was examined by analysing the 18s and 28s ribosomal RNA bands detected and analysed in the Bioanalyzer of the Clinical Hospital of Barcelona (Agilent).

The expression of the analysed genes was measured by means of real-time quantitative RT-PCR normalised with GAPDH. The real-time RT-PCRs were performed in a Bio-Rad Thermal Cycler (iCycler iQ Real-Time PCR Detection System, Bio-Rad, Barcelona) and the amplifications were performed in 20-µl reactions using the two-step RT-PCR Kit with SYBR-Green (Bio-Rad), according to the manufacturer's instructions. The primers were the following: *Ki-*67: forward, SEQ ID NO: 9; reverse, SEQ ID NO: 10; *PCNA*: forward, SEO ID NO: 3; reverse, SEQ ID NO: 4; *GAPDH*: forward, SEQ ID NO: 7; reverse, SEQ ID NO: 8.

### Activity of Caspase 3

The activity of caspase-3 was determined by measuring the proteolysis of the specific substrate (N-acetyl-Asp-Glu-Val-Asp-7-amino-4-methyl coumarin (DEVD-AMC; Biomol, Plymouth Meeting, PA). The cell culture samples were homogenised and sonicated in the assay buffer (50 mM HEPES, 10% sucrose, 0.1 % CHAPS, 5 mM GSSG, 5 mM DTT). The supernatants of the sample were incubated in this buffer, whereto DEVD-AMC at a concentration of 50 µM was added. The AMC released was quantified for 1 hour and a half at 37°C by means of fluorospectrophotometry, using 380 nm of excitation and measuring the emission at 450 nm.

### Statistical analysis

The data are shown as the mean +/- the standard error from the mean (SEM), and values of p>0.05 were considered to be significant. The statistical differences between the groups were analysed by means of variance analysis (ANOVA) and, in the event of significance, Student's t-test was applied.

### RESULTS OBTAINED:

### In vitro analysis of the effect of CAII on renal regeneration

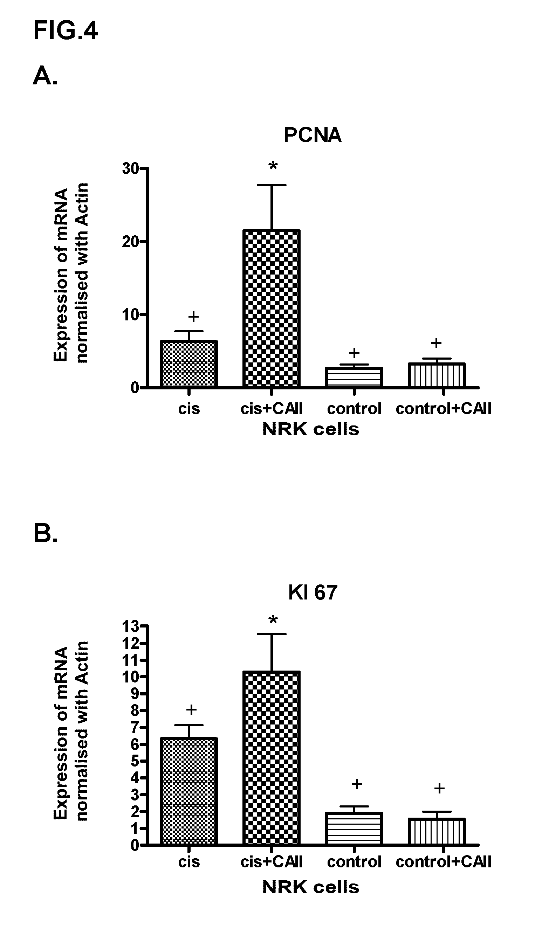

The regeneration study, in this case in the *in vitro* part of our experimental model, is shown in Figure 4. As in the case of the *in vivo* model described in Example 1, this figure shows an analysis of the regeneration marker genes by means of RT-PCR; in this case, PCNA and Ki 67 were selected.

The treatment used in this case to induce cell damage was administration of the drug cisplatin, a known inducer of renal damage. As may be observed in Figure 4, the administration of CAII to cells damaged with cisplatin (group cis + CAII) significantly increased regeneration, shown as an increase in the expression of both PCNA and Ki67.

### In vivo and in vitro analysis of the effect of CAII on apoptosis in renal damage

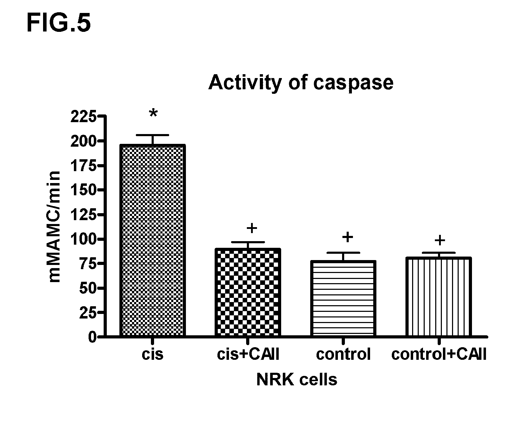

Figure 5 represents the cell damage, determined by the incidence of apoptosis and evaluated by the activity of caspase 3. The *in vitro* part of this study confirms the results obtained *in vivo,* since the administration of CAII to cells treated with cisplatin is capable of reverting apoptosis (cisplatin + CAII).

## Claims

1. Use of a protein which comprises amino acid sequence SEQ ID NO: 1, a variant thereof or a fragment of any of them, for the manufacture of a medicament.

2. Use of a polynucleotide which encodes for the protein, the variant or the fragment according to claim 1, for the manufacture of a medicament.

3. Use of the polynucleotide according to claim 2, which comprises nucleotide sequence SEQ ID NO: 2, for the manufacture of a medicament.

4. Use of a gene construct which comprises the polynucleotide according to any of claims 2 or 3, for the manufacture of a medicament.

5. Use of a vector which comprises the polynucleotide according to any of claims 2 or 3, or the gene construct according to claim 4, for the manufacture of a medicament.

6. Use of a cell which comprises the polynucleotide according to any of claims 2 or 3, the gene construct according to claim 4 or the vector according to claim 5, for the manufacture of a medicament.

7. Use of the protein, the variant or the fragment according to claim 1, the polynucleotide according to any of claims 2 or 3, the gene construct according to claim 4, the vector according to claim 5 or the cell according to claim 6, for the manufacture of a medicament for the prevention and/or treatment of a damage in a tissue or an organ.

8. Use according to claim 7, wherein the damage is caused by ischemia, ischemia-reperfusion or toxins.

9. Use according to claim 8, wherein the toxin is cisplatin.

10. Use according to any of claims 7 to 9, wherein the tissue is an epithelium.

11. Use according to claim 10, wherein the epithelium is selected from the list comprising: renal epithelium, hepatic epithelium, pulmonary epithelium, gastric epithelium, intestinal epithelium, auditory epithelium, pancreatic epithelium, urinary transitional epithelium, uterine epithelium and skin epidermis.

12. Use according to claim 11, wherein the epithelium is the renal epithelium.

13. Use according to any of claims 7 to 9, wherein the organ is selected from the list comprising: kidney, liver, brain, heart, lung, stomach, intestine, ear, pancreas, bladder, uterus and skin.

14. Use according to claim 13, wherein the organ is the kidney.

15. Use of the protein, the variant or the fragment according to claim 1, the polynucleotide according to any of claims 2 or 3, the gene construct according to claim 4, the vector according to claim 5 or the cell according to claim 6, for the manufacture of a medicament for the prevention and/or treatment of an acute failure of an organ caused by ischemia, ischemia-reperfusion or toxins.

16. Use according to claim 15, wherein the toxin is cisplatin.

17. Use of the protein, the variant or the fragment according to claim 1, the polynucleotide according to any of claims 2 or 3, the gene construct according to claim 4, the vector according to claim 5 or the cell according to claim 6, for the manufacture of a medicament for the prevention and/or treatment of the rejection of a transplanted organ.

18. Use according to any of claims 15 to 17, wherein the organ is selected from the list comprising: kidney, liver, brain, heart, lung, stomach, intestine, ear, pancreas, bladder, uterus and skin.

19. Use according to claim 18, wherein the organ is the kidney.

20. Pharmaceutical composition which comprises the protein, the variant or the fragment thereof according to claim 1, the polynucleotide according to any of claims 2 or 3, the gene construct according to claim 4, the vector according to claim 5 or the cell according to claim 6.

21. Pharmaceutical composition according to claim 20, which further comprises a pharmaceutically acceptable vehicle.

22. Pharmaceutical composition according to any of claims 20 or 21, which further comprises another active principle.
